# EUROPÄISCHE PATENTANMELDUNG

(11) **EP 4 014 757 A1**
(43) Veröffentlichungstag der Anmeldung: **22.06.2022**
(21) Anmeldenummer: 21194946.6
(22) Anmeldetag: 05.09.2021
(51) Int. Cl.: A23L 33/105, A23K 40/30, A23P 10/35, A23K 20/111, A23K 20/121

(54) **ZUBEREITUNG ENTHALTEND SILYMARIN, VERFAHREN ZUR HERSTELLUNG UND VERWENDUNG DIESER ZUBEREITUNG**

(30) Priorität: 04.09.2020 DE 102020123115
(71) Anmelder: GITES GmbH, 48336 Sassenberg (DE)
(72) Erfinder: Tünte, Matthias, 48336 Sassenberg (DE); Reeken, Jan-Bernd, 48336 Sassenberg (DE)
(74) Vertreter: Gottfried, Hans-Peter

(57) **Zusammenfassung**

Die Erfindung betrifft eine Zubereitung, enthaltend Silymarin und wenigstens einen Bioenhancer, wobei Silymarin und der Bioenhancer in einer Festphase ko-ausgefällt sind. Weiterhin betrifft die Erfindung ein Verfahren zur Herstellung einer Zubereitung, bei dem Silymarin mit wenigstens einem Bioenhancer vermischt wird. Außerdem betrifft die Erfindung eine Verwendung einer erfindungsgemäßen Zubereitung. Der Erfindung liegt die Aufgabe zugrunde, eine Zubereitung der eingangs genannten Art und ein Verfahren zu deren Herstellung dahingehend zu verbessern, dass möglichst wenig Silymarin eingesetzt werden muss, d.h. eine positive Wirkung des Silymarins unter möglichst geringem und somit ökonomischen Einsatz des Silymarins zu gewährleisten. Diese Aufgabe ist dadurch gelöst, dass als Bioenhancer eine Huminstofffraktion fungiert und die Festphase von einem Fett umkapselt ausgebildet ist. Dies wird im Herstellungsverfahren erreicht, indem als Bioenhancer eine Huminstofffraktionen verwendet wird und nach Vermischung des Silymarins mit dem Bioenhancer ein geschmolzenes Fett zugegeben wird, um eine Suspension zu erhalten, diese Suspension vermengt wird und anschließend die Suspension in einem Sprühkühlverfahren zu einem Granulat verarbeitet wird. Außerdem wird die Aufgabe durch eine Verwendung einer erfindungsgemäßen Zubereitung als Nahrungsergänzungsmittel oder als Futterergänzungsmittel gelöst.

## Beschreibung

Die Erfindung betrifft eine Zubereitung, enthaltend Silymarin und wenigstens einen Bioenhancer, wobei Silymarin und der Bioenhancer in einer Festphase ko-ausgefällt sind. Weiterhin betrifft die Erfindung ein Verfahren zur Herstellung einer Zubereitung, bei dem Silymarin mit wenigstens einem Bioenhancer vermischt wird. Außerdem betrifft die Erfindung eine Verwendung einer erfindungsgemäßen Zubereitung.

Mariendistel mit dem enthaltenen Wirkstoff Silymarin wird bekannterweise zur Behandlung von Leberkrankheiten eingesetzt, da enthaltene Bestandteile des Silymarins, u.a. Silibinin, unter anderem antioxidative Eigenschaften aufweisen (https://de.wikipedia.org/w/index.php?title=Silymarin&oldid=195856229). Ein Bioenhancer ist ein Stoff, der die Aufnahme eines Wirk- oder Nahrungsstoffs in einem Organismus, also einem Lebewesen, verbessert. Dies kann beispielsweise durch eine Optimierung der Löslichkeit, einen verlangsamten oder gehemmten Abbau des Wirk- oder Nahrungsstoffs oder antioxidative Eigenschaften bewirkt werden.

Ko-ausgefällt heißt im Rahmen dieser Schrift, dass zwei Stoffe, konkret Silymarin und Bioenhancer, nebeneinander und bei einem gleichen Schritt ausgefällt werden. Also werden sowohl Silymarin als auch Bioenhancer in ein und demselben Schritt in den festen Zustand überführt, beispielsweise in eine Suspension. Dies heißt insbesondere aber nicht, dass Silymarin die Löslichkeit des Bioenhancers oder umgekehrt beeinflusst. Vielmehr ist ko-ausgefällt im Rahmen dieser Offenbarung so zu verstehen, dass Silymarin und ein Bioenhancer nebeneinander teilweise gelöst vorliegen können und durch Zugabe eines weiteren Stoffes sowohl Silymarin als auch der Bioenhancer ausgefällt werden und somit in einer Suspension vorliegen. Wird diese Suspension weiter verarbeitet, beispielsweise durch Sprühkühlen, erhält man also einen Feststoff, der sowohl Silymarin als auch den Bioenhancer enthält.

Aus der Veröffentlichung D. Tedesco et al., J. Dairy Sci., 2004, 87, 2239-2247 ist bekannt, Silymarin als Nahrungsergänzungsmittel an Kühe zu verfüttern, um deren Milchleistung zu steigern. Hierfür wurden die Kühe mit 10 g unbearbeitetem Silymarin täglich ergänzend zu ihrem regulären Futter gefüttert. Dies führte zu einer erhöhten Milchleistung gegenüber einer Kontrollgruppe.

Aus der Veröffentlichung Javed et al., Clinical Phytoscience, 2018, 4, 18-26 ist außerdem bekannt, dass die Aufnahme von Silymarin in Säugetieren durch Zugabe von Bioenhancern verbessert werden kann, wobei als Bioenhancer Lysergol, Piperin oder Fulvosäure genannt werden.

Mariendistelextrakt beinhaltet Silymarin, welches regelmäßig bis zu etwa 40 bis 65 % Silibinin und 10 bis 20 % Isosilibinin enthält. Diese Werte schwanken für einen Extrakt aus einem Naturpräparat jedoch. Für einen wirkungsvollen Einsatz sollten bei einer HPLC-Analyse des Silymarins wenigstens 30% als Summe aus Silibinin und Isosilibinin feststellbar sein.

Der Erfindung liegt die Aufgabe zugrunde, eine Zubereitung der eingangs genannten Art und ein Verfahren zu deren Herstellung dahingehend zu verbessern, dass möglichst wenig Silymarin eingesetzt werden muss, d.h. eine positive Wirkung des Silymarins unter einem möglichst geringen und somit ökonomischem Einsatz des Silymarins zu gewährleisten.

Diese Aufgabe ist dadurch gelöst, dass als Bioenhancer eine Huminstofffraktion fungiert und die Festphase von einem Fett umkapselt ausgebildet ist. Dies wird im Herstellungsverfahren erreicht, indem als Bioenhancer eine Huminstofffraktionen verwendet wird und nach Vermischung des Silymarins mit dem Bioenhancer ein geschmolzenes Fett zugegeben wird, um eine Suspension zu erhalten, diese Suspension vermengt wird und anschließend die Suspension in einem Sprühkühlverfahren zu einem Granulat verarbeitet wird. Erfindungsgemäß kann eine so hergestellte Zubereitung vorteilhaft Verwendung als Futterergänzungsmittel oder als Nahrungsergänzungmittel finden.

Vermengen ist hierbei als ein Synonym zu vermischen, mischen und dispergieren zu verstehen.

Mit anderen Worten ist das Silymarin einerseits mit einem Bioenhancer vermengt, und andererseits ist die aus Silymarin und Bioenhancer gebildete Festphase von einem Fett umkapselt. Diese Umkapselung führt dazu, dass bei Aufnahme der Zubereitung mit der Nahrung die Zubereitung in einem Magen oder Pansen von dem Fett geschützt wird und das Silymarin dort nicht in erheblichem Maße frühzeitig abgebaut werden kann. Somit kommt ein großer Anteil des eingesetzten Silymarins in einem Darmtrakt eines jeweiligen Lebewesens an und kann dort in den Blutkreislauf aufgenommen werden. Somit wird die Bioverfügbarkeit des Silymarins einerseits durch den Bioenhancer gesteigert, andererseits aber ebenso durch das Fett.

Erfindungsgemäß haben sich als Bioenhancer insbesondere Olivenextrakt sowie Huminstofffraktionen als vorteilhaft herausgestellt. Weiterhin können auch Quercetin, Alinin, Piperin, Glyzyrrhizin, Cynarin sowie weitere Stoffe, die im Stand der Technik als Bioenhancer bekannt sind, verwendet werden. Gemäß der Erfindung kann das Silymarin in der Festphase und somit resultierend in dem Granulat mit genau einem der Bioenhancer kombiniert werden, es ist jedoch auch Bestandteil der Erfindung, Silymarin mit zwei oder mehreren Bioenhancern in der Festphase und somit im Granulat zu verwenden.

Das Sprühkühlverfahren ist ein etablierter Prozess zur Herstellung von Granulaten und Pulvern. Die Suspension der Festphase in dem geschmolzenen Fett wird durch einen Zerstäuber oder eine Druckdüse in eine Kühlkammer geleitet, wo eine Art Sprühnebel entsteht, welcher abgekühlt wird und sich so homogene Granulatpartikel bilden. Dies ermöglicht eine besonders gleichmäßige Verteilung des Silymarins und des Bioenhancers im Granulat. Außerdem kann durch die Bildung des Granulats eine Staubbildung und somit ein Verlust des Silymarins vermieden werden. Vorteilhaft weist das erhaltene Granulat zu 99% eine Korngröße von weniger als 1500 µm auf.

Andere vorteilhafte Weiterbildungen ergeben sich aus den abhängigen Patentansprüchen.

Gemäß einer vorteilhaften Weiterbildung der Erfindung liegen das Silymarin und der Bioenhancer in der Festphase in einem Massenverhältnis von 5:1 bis 0,5:1 vor. Besonders bevorzugt liegt das Massenverhältnis der Festphase zu dem Fett bei 1:3 bis 1:5, ganz besonders bevorzugt bei 1:4. Dies verdeutlicht, dass der Hauptbestandteil des Granulats das Fett ist, welches mit der Festphase angereichert ist. Dies ermöglicht einen besonders sparsamen Einsatz des Silymarins.

Erfindungsgemäß ist das Fett ein pflanzliches Fett, zum Beispiel Palm- oder Rapsfett. Dabei werden vorzugsweise gehärtete und raffinierte Fette verwendet. Diese sind in einem sauren Milieu, wie in einem Magen oder Pansen, stabil und eine Reaktion mit anderen Wirk- oder Nahrungsstoffen kann ausgeschlossen werden. Der Schmelzpunkt des zu verwendenden Fettes liegt in einem Bereich von 50 ― 70°C.

Vorteilhaft liegt die von Fett umkapselte Festphase als Granulat vor. Dies ermöglicht eine einfache Handhabung und Abmessbarkeit der Zubereitung.

In einer Weiterbildung der Erfindung weist das Granulat einen Schmelzpunkt größer als 60°C auf. Somit schmilzt das Granulat nicht in einem Organismus bzw. Lebewesen. Weiterhin weist das Granulat bei 20°C eine Dichte von 450-500 g/l auf.

Erfindungsgemäß werden das Silymarin und der Bioenhancer jeweils zu 99% mit einer Korngröße von weniger als 300 µm eingesetzt. Dies ermöglicht eine effiziente Durchführung des Verfahrens, da das Silymarin und der Bioenhancer exzellent vermischt werden können.

Besonders bevorzugt wird das geschmolzene Fett mit einer Temperatur von maximal 90°C hinzugegeben, die Festphase aus Silymarin und Bioenhancer wird also in geschmolzenem Fett mit maximal 90°C schonend vermengt, sodass keine Degradation des Silymarins erfolgt.

Gemäß einer Weiterbildung des erfindungsgemäßen Verfahrens wird die Suspension mindestens 5 Minuten lang vermengt. Dies führt zu einer besonders homogenen Verteilung der Festphase in der Suspension und somit zu einer homogenen Verteilung des Silymarins und des Bioenhancers im Granulat.

Die erfindungsgemäße Zubereitung kann beispielsweise als Futterergänzungsmittel, also eine Ergänzung des Futtermittels für Nutztiere, insbesondere Warmblüter, eingesetzt werden. Bei einer Verwendung bei Milchkühen zur Erhöhung der Milchleistung kann beispielsweise festgestellt werden, dass bei täglicher Fütterung von 4 g des Granulats, in dem etwa 0,8 g Silymarin enthalten sind, eine äquivalente Steigerung der Milchleistung beobachtet werden kann wie bei einer täglichen Fütterung von 10 g unbearbeiteten Silymarins, jeweils in Ergänzung zu regulärem Futtermittel. Somit führt die erfindungsgemäße Zubereitung durch eine gesteigerte Bioverfügbarkeit zu erheblichen Kosten- und Ressourceneinsparungen.

Die erfindungsgemäße Zubereitung kann ebenso vorteilhaft als Nahrungsergänzungsmittel verwendet werden, beispielsweise zur Behandlung von Leberkrankheiten bei Menschen.

Das Granulat kann beispielhaft folgende Zusammensetzungen aufweisen:
- Silymarin (Mariendistelextrakt) 16m-%, Olivenextrakt 4m-%, Fett 80m-%,
- Silymarin (Mariendistelextrakt) 10m-%, Huminstoff 10m-%, Fett 80m-%,
- Silymarin (Mariendistelextrakt) 12m-%, Olivenextrakt 4m-%, Huminstoff 4m-%, Fett 80m-%.

Die genannten Bestandteile sind bezogen auf die Masse des Granulats, m-% bedeutet also Masseprozent. Es wird noch einmal deutlich, dass als Bioenhancer ein o.g. Stoff eingesetzt werden kann, jedoch auch eine Kombination verschiedener Bioenhancer möglich ist.

## Patentansprüche

1. Zubereitung, enthaltend Silymarin und wenigstens einen Bioenhancer, wobei Silymarin und der Bioenhancer in einer Festphase ko-ausgefällt sind, **dadurch gekennzeichnet, dass** als Bioenhancer eine Huminstofffraktion fungiert und die Festphase von einem Fett umkapselt ausgebildet ist.

2. Zubereitung nach Anspruch 1, **dadurch gekennzeichnet, dass** das Silymarin und der Bioenhancer in der Festphase in einem Massenverhältnis von 5:1 bis 0,5:1 vorliegen.

3. Zubereitung nach Anspruch 1 oder 2, **dadurch gekennzeichnet, dass** das Fett ein pflanzliches Fett ist.

4. Zubereitung nach einem der vorhergehenden Ansprüche, **dadurch gekennzeichnet, dass** die von Fett umkapselte Festphase als Granulat vorliegt.

5. Zubereitung nach Anspruch 4, **dadurch gekennzeichnet, dass** das Granulat einen Schmelzpunkt größer als 60°C aufweist.

6. Zubereitung nach Anspruch 4 oder 5, **dadurch gekennzeichnet, dass** das Granulat bei 20°C eine Dichte von 450-500 g/l aufweist.

7. Verfahren zur Herstellung einer Zubereitung, bei dem Silymarin mit wenigstens einem Bioenhancer vermischt wird, **dadurch gekennzeichnet, dass** als Bioenhancer eine Huminstofffraktionen verwendet wird und nach Vermischung des Silymarins mit dem Bioenhancer ein geschmolzenes Fett zugegeben wird, um eine Suspension zu erhalten, diese Suspension vermengt wird und anschließend die Suspension in einem Sprühkühlverfahren zu einem Granulat verarbeitet wird.

8. Verfahren nach Anspruch 7, **dadurch gekennzeichnet, dass** das geschmolzene Fett mit einer Temperatur von maximal 90°C hinzugegeben wird.

9. Verfahren nach Anspruch 7 oder 8, **dadurch gekennzeichnet, dass** die Suspension mindestens 5 Minuten lang vermengt wird.

10. Verfahren nach einem der Ansprüche 7 bis 9, **dadurch gekennzeichnet, dass** das Silymarin und der Bioenhancer jeweils zu 99% mit einer Korngröße von weniger als 300 µm eingesetzt werden.

11. Verwendung einer Zubereitung, enthaltend Silymarin und wenigstens einen Bioenhancer, wobei Silymarin und der Bioenhancer in einer Festphase ko-ausgefällt sind und die Festphase von einem Fett umkapselt ausgebildet ist, als Nahrungsergänzungsmittel oder als Futterergänzungsmittel, wobei als Bioenhancer eine Huminstofffraktion fungiert.
